(19) Europäisches Patentamt
European Patent Office
Office européen des brevets

(11) Veröffentlichungsnummer: **0 376 047 B1**

(12) # EUROPÄISCHE PATENTSCHRIFT

(45) Veröffentlichungstag der Patentschrift: **04.05.94**

(51) Int. Cl.$^5$: **C09B 51/00**, A61K 7/13

(21) Anmeldenummer: **89122937.9**

(22) Anmeldetag: **12.12.89**

Die Akte enthält technische Angaben, die nach dem Eingang der Anmeldung eingereicht wurden und die nicht in dieser Patentschrift enthalten sind.

(54) **2-Amino-6-chlor-4-nitro-phenolderivate, Verfahren zu ihrer Herstellung und Haarfärbemittel mit einem Gehalt an diesen Verbindungen.**

(30) Priorität: **23.12.88 DE 3843578**

(43) Veröffentlichungstag der Anmeldung:
**04.07.90 Patentblatt 90/27**

(45) Bekanntmachung des Hinweises auf die Patenterteilung:
**04.05.94 Patentblatt 94/18**

(84) Benannte Vertragsstaaten:
**DE ES FR GB IT**

(56) Entgegenhaltungen:
**FR-A- 2 180 651**
**FR-A- 2 540 106**
**GB-A- 486 086**

(73) Patentinhaber: **Wella Aktiengesellschaft**
**Berliner Allee 65**
**D-64295 Darmstadt(DE)**

(72) Erfinder: **Mager, Herbert, Dr.**
**Route du Roule 21**
**CH-1723 Marly(CH)**
Erfinder: **Braun, Hans-Jürgen, Dr.**
**Kapellacker 10 D**
**CH-3182 Überstorf(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Gegenstand der Erfindung sind bestimmte neue 2-Amino-6-chlor-4-nitro-phenolderivate, Verfahren zu Ihrer Herstellung sowie Haarfärbemittel welche als direktfärbenden Nitrofarbstoffe 2-Amino-6-chlor-4-nitro-phenolderivate enthalten.

Nitrofarbstoffe haben für die Haarfärbung eine wesentliche Bedeutung erlangt. Durch Kombination verschiedener Nitrofarbstoffe lassen sich Haarfärbemittel herstellen, die ohne Zusatz von Oxidationsmitteln Haare in natürlichen oder in modischen Tönen zu färben vermögen. Die Nitrofarbstoffe sind auch wichtige Bestandteile von Oxidationshaarfärbemitteln in denen sie zur Abrundung des Farbergebnisses und zur Erzeugung von modischen Effekten herangezogen werden.

Zur Erzeugung von modischen Roteffekten verden insbesondere solche Nitrofarbstoffe benötigt, welche dem Haar eine intensive Rotfärbung verleihen können.

Für diese Aufgabe wurde lange Zeit das 2-Nitro-p-phenylendiamin verwendet, gegen das in letzter Zeit toxikologische Bedenken erhoben worden sind.

An Farbstoffe, die zum Färben von menschlichen Haaren verwendet werden sollen, werden eine ganze Reihe von Anforderungen gestellt. Sie müssen in toxikologischer und dermatologischer Hinsicht unbedenklich sein.

Außerdem wird für die Haarfärbung eine gute Licht-. Säure- und Reibeechtheit gefordert. Ihre Verwendung in Oxidationshaarfärbemitteln setzt weiterhin voraus, daß die Nitrofarbstoffe gegenüber Wasserstoffperoxid in ammoniakalischer Lösung und gegenüber Antioxidantien stabil sind.

Ein Nitrofarbstoff, der als Zusatz zu oxidativen Haarfärbemitteln geeignet sein soll, muß auch den im Vergleich zu den Haarspitzen weniger geschädigten Haaransatz in ausreichender Intensität anfärben. Die Erfüllung dieser Forderung ist sehr wichtig für die Erzielung eines ausgeglichenen Farbergebnisses, beispielsweise bei den üblicherweise häufig verwendeten Kuppler-Entwicklersubstanz-Kombinationen 4-Aminophenol/2,4-Diaminoanisol oder 4-Amino-3-methyl-phenol/2,4-Diamino-anisol. Bei einem Färbeversuch werden mit einer solchen Kombination die stärker geschädigten Haarspitzen stärker rot angefärbt als die weniger geschädigten Haarwurzeln. Zum Ausgleich dieses Färbeverhaltens müssen Haarfärbemitteln Nitrofarbstoffe zugesetzt werden, die bevorzugt den Haaransatz anfärben.

Ein Beispiel für einen solchen Nitrofarbstoff ist das 2-Nitro-1,4-diaminobenzol. Der Farbstoff färbt das Haar in einem intensiven neutralen Rot. Er hat aber den Nachteil, daß er nicht gut lagerbeständig ist, insbesondere in Gegenwart von Reduktionsmitteln, wie zum Beispiel Ascorbinsäure. Ausserdem ist 2-Nitro-1,4-diaminobenzol mutagen.

Weiterhin sind aus der FR-A 2 540 106 6-Chlor-3-nitro-4-aminophenolderivate bekannt, die für die Verwendung in Haarfärbemitteln geeignet sein sollen.

Diese Verbindungen sind Jedoch nach den in dieser Schrift angegebenen Herstellungsverfahren nur teilweise herstellbar und zudem bezüglich ihrer färberischen Eigenschaften sowie ihrer Lagerstabilität nicht zufriedenstellend. So liefern die dort beschriebenen 6-Chlor-3-nitro-4-hydroxyalkylaminophenole sowie die 6-Chlor-3-nitro-4-alkylaminophenole lediglich orange Töne beziehungsweise farbschwache Selbsttöne, nicht jedoch die gewünschten farbintensiven neutralen Rottöne.

Zudem sind diese Verbindungen in den Haarfärbemitteln nur für einen begrenzten Zeitraum haltbar, wodurch die Lagerfähigkeit der Haarfärbemittel erheblich eingeschränkt wird.

Es bestand daher die Aufgabe, einen direktfärbenden Haarfarbstoff zur Verfügung zu stellen, der das Haar in einem intensiven, reinen Rotton zu färben vermag, eine gute Lagerfähigkeit besitzt, und dabei sowohl physiologisch besser verträglich als auch in färberischer Hinsicht vorteilhafter als die aus dem Stand der Technik bekannten Haarfarbstoffe ist.

Es wurde nun gefunden, daß durch ein 2-Amino-6-chlor-4-nitro-phenolderivat der allgemeinen Formel (I)

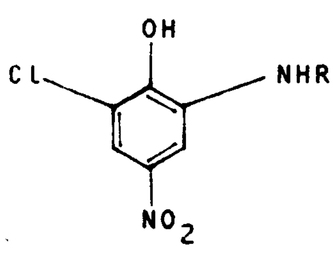

(I),

worin der Rest R einen geradkettigen oder verzweigten Alkylrest mit 1 bis 5 Kohlenstoffatomen bedeutet, die gestellte Aufgabe in hervorragender Weise gelöst wird.

Beispiele für erfindungsgemäße Verbindungen der Formel (I) sind 2-Chlor-6-methylamino-4-nitro-phenol, 2-Chlor-6-ethylamino-4-nitro-phenol, 2-Chlor-4-nitro-6-propylaminophenol, 2-Chlor-6-((2′-methylpropyl)-amino)-4-nitro-phenol und 2-Chlor-6-( (2′,2′-dimethylpropyl)amino)-4-nitrophenol.

Die neuen Nitrofarbstoffe gemäß der allgemeinen Formel (I) können beispielsweise durch die folgenden Verfahren hergestellt werden:

Nach einem ersten Verfahren setzt man zunächst 2-Amino-6-chlor-4-nitro-phenol mit einem Carbonsäurechlorid der Formel $R^1C(O)Cl$ oder einem Carbonsäureanhydrid der Formel $O(C(O)R^1)_2$ , wobei $R^1$ Wasserstoff oder einen geradkettigen oder verzweiten Alkylrest mit 1 bis 4 Kohlenstoffatomen darstellt, um und reduziert sodann das entstandene, mit $R^1$ substituierte N-(3-Chlor-2-hydroxy-5-nitrophenyl)carbonsäureamid mit Natriumborhydrid in Gegenwart von Bortrifluoridetherat zu der Verbindung der Formel (I). Der Reduktionsschritt wird hierbei vorzugsweise bei 60 Grad Celsius in Tetrahydrofuran als Lösungsmittel durchgeführt.

Nach einem zweiten Verfahren löst man zunächst 2-Amino-6-chlor-4-nitro-phenol in einer Carbonsäure der Formel $R^1C(O)OH$, worin $R^1$ Wasserstoff oder einen geradkettigen oder verzweigten Alkylrest mit 1 bis 4 Kohlenstoffatomen bedeutet, versetzt sodann die so erhaltene Lösung mit Natriumborhydrid und erhitzt anschließend das Reaktionsgemisch auf 60 bis 70 Grad Celsius. Das gewünschte Alkylierungsprodukt kann sehr einfach durch Verdünnen des Reaktionsgemisches mit Wasser isoliert werden.

Nach einem dritten Verfahren setzt man zunächst 2-Amino-6-chlor-4-nitro-phenol mit einem Orthocarbonsäurester der Formel $R^1C(OR^2)_3$, worin $R^1$ Wasserstoff oder einen geradkettigen oder verzweigten Alkylrest mit 1 bis 4 Kohlenstoffatomen darstellt und $R^2$ Methyl oder Ethyl bedeutet, um und reduziert das so erhaltene Benzoxazolderivat sodann mit Natriumborhydrid in alkoholischer Lösung.

Als geeignete Orthocarbonsäureester kommen beispielsweise der Orthoameisensäuretriethylester, der Orthoameisensäuretrimethylester und der Orthoessigsäuretriethylester in Betracht.

Die neuen Nitrofarbstoffe der Formel (I) weisen vorteilhafte überraschende Eigenschaften auf.

Nach dem Stand der Technik kann der Farbton eines Aminonitrophenols nach Rot verschoben werden, indem dieses Aminonitrophenol am Aminostickstoff mit einer Hydroxyalkylgruppe substituiert wird. Es wurde jedoch gefunden, daß die Substitution der Aminogruppe im 2-Amino-6-chlor-4-nitro-phenol mit einer 2-Hydroxyethylgruppe nur eine minimale Farbverschiebung bewirkt. Dagegen wurde überraschend gefunden, daß bei Substitution der Aminogruppe im 2-Amino-6-chlor-4-nitro-phenol mit einer Alkylgruppe Farbstoffe erhalten werden, die das Haar in tiefen, intensiven Rottönen zu färben vermögen.

Darüberhinaus weisen die Verbindungen der Formel (I) in physiologischer Hinsicht bessere Eigenschaften auf, als aus dem Stand der Technik bekannte rotfärbende Nitrofarbstoffe. So zeigt beispielsweise die Verbindung 2-Chlor-6-ethylamino-4-nitro-phenol in einem Mutagenitätstest nach Ames am Salmonella typhimurium Stamm TA 98, keine Mutagenität, während 2-Nitro-1,4-diaminobenzol mutagen ist.

Die neuen Verbindungen der Formel (1) besitzen auch überraschende anwendungstechnische Vorteile. Der Haarfärbefachmann erwartet, daß bei einer Vergrößerung eines Farbstoffmoleküls und bei einer verschlechterten Wasserlöslichkeit die Intensität der erzielbaren Haarfärbung verringert wird. Es wurde jedoch überraschend gefunden, daß, obwohl die Wasserlöslichkeit bei der Substitution des Grundkörpers 2-Amino-6-chlor-4-nitro-phenol an der Aminogruppe mit zunehmender Größe des Substituenten abnimmt, die Intensität der erzielten Haarfärbung nicht abnimmt.

Weiterhin wurde bei Färbeversuchen mit den erfindungsgemäßen Farbstoffen, beispielsweise mit 2-Chlor-6-((2′-methylpropyl)amino-4-nitro-phenol, gefunden, daß der Haaransatz in einem leuchtend roten Farbton gefärbt wird. Ein solches Färbeergebnis war bisher nur mit dem 2-Nitro-p-phenylendiamin zur erhalten. Die neuen Verbindungen gemäß der allgemeinen Formel (I) stellen daher einen wesentlichen Fortschritt in der Haarfärbetechnik dar, da es nun möglich ist, das toxikologisch bedenkliche 2-Nitro-p-phenylendiamin durch eine fachlich gleichwertige, aber in toxikologischer Hinsicht verbesserte Verbindung zu ersetzen.

Oxidativen Haarfärbemitteln, welche als Farbstoffvorläufer, beispielsweise eine Kombination aus p-Phenylendiamin, p-Aminophenol oder deren Derivaten mit metasubstituierten Benzolderivaten wie Resorcin oder m-Phenylendiaminderivaten enthalten, müssen zur Vermeidung einer unerwünschten Dunkelfärbung der Färbemasse während der Produktion, Abfüllung und Lagerung Antioxidantien, wie Ascorbinsäure oder Natriumsulfit, zugesetzt werden. Diese Antioxidantien sind, wie auch die in den oxidativen Haarfärbemitteln eingesetzten p-Phenylendiamin- oder p-Aminophenolderivate Reduktionsmittel, welche imstande sind, gewisse Nitroverbindungen zu reduzieren.

So ist jedem Haarfärbefachmann geläufig, daß das 2-Nitro-p-phenylendiamin in oxidativen Haarfäbemitteln langsam abgebaut wird. Die Farbnuance, die mit einem solchen Haarfärbemittel erhalten wird, ist nach

einer Lagerdauer von 3-6 Monaten gegenüber einem neuen Ansatz deutlich verschoben. Dieses unerwünschte Verhalten kann überraschenderwei se vermieden werden, wenn anstelle des 2-Nitro-p-phenylendiamins als Rotkomponente die neuen Verbindungen gemäß der allgemeinen Formel (I) eingesetzt werden. Die Lagerstabilität eines Haarfärbemittel mit einem Gehalt an den neuen roten Nitrofarbstoffen ist deutlich verbessert gegenüber den üblichen, dem Stand der Technik entsprechenden Formulierungen.

Durch die Verbindung der Formel (I) kann das Nitro-p-phenylendiamin in Haarfärbemitteln vollwertig und vorteilhaft ersetzt werden.

Gegenstand der vorliegenden Anmeldung ist daher auch ein Mittel zur Färbung von Haaren mit einem Farbstoffgehalt und für Haarfärbemittel üblichen Zusätzen, dadurch gekennzeichnet, daß es 2-Amino-6-chlor-4-nitro-phenol-derivate der allgemeinen Formel (I)

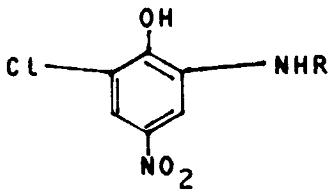

(I),

worin der Rest R eine geradkettigen oder verzweigten Alkylrest mit 1 bis 5 Kohlenstoffatomen bedeutet, oder dessen physiologisch verträgliches wasserlösliches Salz enthält.

Das erfindungsgemäße Mittel zur Färbung von Haaren betrifft sowohl eine Ausführungsform, bei der es ohne Zugabe eines Oxidationsmittels angewendet wird, als auch eine weitere Ausführungsform, bei der die Zugabe eines Oxidationsmittels erforderlich ist.

Bei dem ersten Haarfärbemittel ohne Oxidationsmittelzugabe handelt es sich um ein Mittel, das neben den Farbstoffen der angegebenen Formel (I) noch andere bekannte direktfärbende Haarfarbstoffe enthalten kann. Von diesen für die Haarfärbung bekannten Farbstoffen seien beipielsweise die folgenden erwähnt: Aromatische Nitrofarbstoffe, wie zum Beispiel 2-Amino-4-nitro-phenol, 1-(2′-Hydroxyethyl)amino-2-amino-4-nitro-benzol, 2-Nitro-4-(2′-hydroxyethyl)amino-anilin, 1-Methylamino-2-nitro-4-di-(2′-hydroxyethyl)-amino-benzol, 1-(2′,3′-Dihydroxypropyl)amino-2-nitro-4-(N-ethyl,N-(2″-hydroxyethyl)amino)-benzol, 1-(2′,3′-Dihydroxypropyl)amino-2-nitro-4-dimethylamino-benzol, 1-(2′,3′-Dihydroxypropyl)amino-2-nitro-4-pyrrolidino-benzol, 1-(3′-Hydroxypropyl)amino-2-nitro-4-di-(2″-hydroxyethyl)amino-benzol, 2,5-Bis(2′-hydroxyethyl)amino-nitro-benzol, Triphenylmethanfarbstoffe wie zum Beispiel Basic Violet 1 (C.I. 42 535), Azofarbstoffe, wie zum Beispiel Acid Brown 4 (C.I. 14 805), Disperse Violet 4 (C.I. 61 105), Antrachinonfarbstoffe, wie zum Beispiel 1,4,5,8-Tetraamino-anthrachinon oder 1,4-Diamino-anthrachinon, wobei die Farbstoffe dieser Klassen je nach Art ihrer Substituenten sauren, nichtionogenen oder basischen Charakter haben können. Weitere geeignete direkt auf das Haar aufziehende Farstoffe sind beispielsweise in dem Buch von J.C. Johnson, "Hair Dyes" Noyes Data Corp. Park-Ridge (USA) (1973), Seiten 3 bis 91 und 113 bis 139, beschrieben.

Die Zübereitungsform des hier beschriebenen Haarfärbemittels auf der Basis von direktfärbenden Farbstoffen kann beispielsweise ein Lösung, insbesondere ein wäßrigalkoholische Lösung, sein. Bevorzugte Zübereitungsformen sind weiterhin eine Creme, ein Gel oder eine Emulsion. Ebenfalls ist es möglich, dieses Mittel mit Hilfe eines Zerstäubers beziehungsweise anderer geeigneter Pump- oder Sprühvorrichtungen oder im Gemisch mit üblichen unter Druck verflüssigten Treibmitteln als Aerosolspray oder Aerosolschaum aus einem Druckbehälter zu entnehmen.

Die Farbstoffe der angegebenen Formel (I) sollen in diesen Färbemitteln ohne Oxidationsmitel-Zugabe in einer Konzentration von etwa 0,01 bis 2,0 Gewichtsprozent, vorzugsweise von 0,01 bis 1,0 Gewichtsprozent, enthalten sein. Der Gesamtgehalt an direktfärbenden Farbstoffen liegt in den Grenzen von etwa 0,01 bis 3,0 Gewichtsprozent.

Der pH-Wert dieses Färbemittels liegt im Bereich von 3 bis 12, insbesondere bei pH 8 bis 11,5 wobei die Einstellung des gewünschten alkalischen pH-Wertes vorzugsweise mit Ammoniak erfolgt, jedoch auch mit organischen Aminen wie beispielsweise Monoethanolamin oder Triethanolamin vorgenommen werden kann.

Die Anwendung dieses Färbemittels erfolgt in üblicher Weise durch Aufbringen einer für die Haarfärbung ausreichenden Menge des Mittels auf die Haare, mit denen es eine Zeitlang, etwa 5 bis 10 Minuten, in Berührung bleibt. Anschließend wird das Haar mit Wasser, gegebenenfalls noch mit einer wäßrigen Lösung einer schwachen organischen Säure, gespült und getrocknet. Als schwache organische Säure können beispielsweise Essigsäure, Zitronensäure oder Weinsäure Verwendung finden.

Das vorstehend beschriebene Haarfärbemittel ohne Oxidationsmittelzugabe kann selbstverständlich auch in der Kosmetik üblicherweise verwendete synthetische natürliche oder modifizierte natürliche Polymere enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, basische Polymerisate von Estern von Polyacrylsäure, Polymethylacrylsäure und Aminoalkoholen beziehungsweise deren Salze oder Quaternisierungsprodukte, Polyacrylnitril, Polyvinylacetate sowie Copolymerisate aus derartigen Verbindungen wie Polyvinylpyrrolidon-Vinylacetat und dergleichen erwähnt.

Auch natürliche Polymere oder modifizierte natürliche Polymere wie Chitosan (entacetyliertes Chitin) oder Chitosanderivate, können für den genannten Zweck eingesetzt werden.

Die kosmetischen Polymere sind in diesem Mittel in der für solche Mittel üblichen Menge von etwa 1 bis 5 Gewichtsprozent enthalten. Der pH-Wert des Mittels liegt im Bereich von etwa 6,0 bis 9,0.

Die Anwendung dieses Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich kann das vorstehend beschriebene Haarfärbemittel ohne Oxidationsmittel-Zugabe gebenenfalls weitere, für Haarfärbemittel übliche Zusätze, wie zum Beispiel Pflegestoffe, Netzmittel, Verdicker, Weichmacher, Konservierungsstoffe und Parfümöle sowie auch andere, nachstehend für Oxidationshaarfärbemittel aufgeführte übliche Zusätze enthalten.

Zum Gegenstand der vorliegenen Erfindung gehört auch, wie eingangs erwähnt, ein Haarfäbemittel, bei dem die Zugabe eines Oxidationsmittels erforderlich ist. Es enthält außer den Farbstoffen gemäß der angegebenen Formel (I) und gegebenenfalls bekannten, direkt auf das Haar aufziehenden Farbstoffen noch zusätzliche, bekannte Oxidationsfarbstoffe, die einer oxidativen Entwicklung bedürfen.

Bei diesen Oxidationsfarbstoffen handelt es sich hauptsächlich um aromatische p-Diamine und p-Aminophenole, wie beispielsweise p-Toluylendiamin, p-Phenylendiamin, p-Aminophenol und ähnliche Verbindungen, die zum Zwecke der Nuancierung der Färbung mit sogenannten Modifiern, wie zum Beispiel m-Phenylendiamin, Resorcin, m-Aminophenol und anderen, kombiniert werden.

Derartige zur Haarfärbung bekannte und übliche Oxidationsfarbstoffe sind unter anderem in dem Buch von E. Sagarin, "Cosmetics, Science and Technology" (1957), Interscience Publishers Inc., New York, Seiten 503 ff. sowie in dem Buch von H. Janistyn, "Handbuch der Kosmetika und Riechstoffe" (1973), Seiten 338 ff., beschrieben.

Mit Mischungen aus diesen Oxidationsfarbstoffen und den Farbstoffen gemäß der angegebenen Formel (I) lassen sich sehr gut natürliche Blond- und Brauntöne, aber auch modische Nuancen herstellen.

Die Farbstoffe gemäß der Formel (I) sind in diesem Färbemittel mit Oxidationsmittel-Zugabe in einer Konzentration von etwa 0,01 bis 4,0 Gewichtsprozent, vorzugsweise 0,02 bis 2,0 Gewichtsprozent, enthalten. Der Gesamtgehalt an Farbstoffen in diesem Färbemittel beträgt etwa 0,1 bis 5,0 Gewichtsprozent.

Oxidationshaarfärbemittel sind im allgemeinen alkalisch eingestellt, vorzugsweise auf pH-Werte von etwa 8,0 bis 11,5, wobei die Einstellung überwiegend mit Ammoniak erfolgt. Es können dazu aber auch andere organische Amine, zum Beispiel Monoethanolamin oder Triethanolamin, verwendet werden. Als Oxidationsmittel zur Entwicklung der Haarfärbung kommen hauptsächlich Wasserstoffperoxid und dessen Additionsverbindungen in Betracht. Die Zübereitungsform dieses Haarfärbemittels kann die gleiche wie bei dem Haarfärbemittel ohne Oxidationsmittel-Zugabe sein. Vorzugsweise ist sie die einer Creme oder eines Gels.

Übliche Zusätze in Cremes, Emulsionen oder Gelen sind zum Beispiel Lösungsmittel wie Wasser, niedere aliphatische Alkohole, beispielsweise Ethanol, Propanol, Isopropanol, Glycerin oder Glykole, wie zum Beispiel Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen, wie zum Beispiel Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker, wie zum Beispiel höhere Fettalkohole, Bentonit, Stärke, Polyacrylsäure, ferner Cellulosederivate, wie zum Beispiel Carboxymethylcellulose, Alginate, Vaseline, Paraffinöl und Fettsäuren sowie außerdem Pflegestoffe, wie zum Beispiel Lanolinderivate, Cholesterin, Pantothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentratinen von etwa 0,5 bis 30 Gewichtsprozent, während die Verdicker in einer Menge von etwa 0,1 bis 5 Gewichtsprozent in den Zübereitungen enthalten sein können.

Die Anwendung der genannten Zübereitung, bei der die Zugabe eines Oxidationsmittels erforderlich ist, erfolgt in bekannter Weise, indem man das Haarfärbemittel vor der Behandlung mit dem Oxidationsmittel vermischt und eine zur Färbung des Haares ausreichende Menge der Mischung, im allgemeinen etwa 50 bis 150 ml, auf das Haar aufträgt. Nach einer für die Haarfärbung ausreichenden Einwirkungszeit, die üblicherweise etwa 10 bis 45 Minuten beträgt, wird das Haar mit Wasser, gegebenenfalls anschließend mit der wäßrigen Lösung einer schwachen organischen Säure, wie zum Beispiel Zitronensäure oder Weinsäure, gespült und sodann getrocknet.

Hinsichtlich der färberischen Möglichkeiten bietet das erfindungsgemäße Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt. Hierbei wird das Färbemittel je nach Zusammensetzung entweder in Verbindung mit Wasserstoffperoxid oder auch ohne Oxidationsmittel angewandt.

Die nachstehenden Beispiele sollen den Anmeldungsgegenstand erläutern, ohne ihn auf die Beispiele zu beschränken.

**HERSTELLUNGSBEISPIELE**

**Beispiel 1:**

Herstellung von 2-Chlor-6-ethylamino-4-nitro-phenol

18

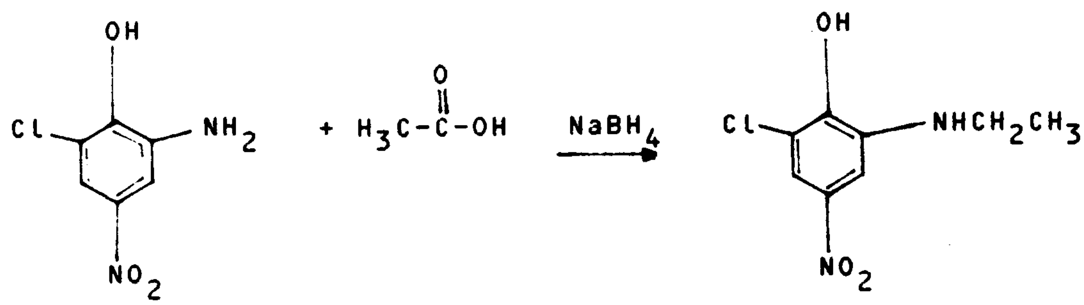


$C_6H_5ClN_2O_3$ (188,54) $C_2H_4O_2$ (64,08) (37,83) $C_8H_9ClN_2O_3$ (216,6)

3,77 g (20 mmol) 2-Amino-6-chlor-4-nitro-phenol werden in 100 ml Essigsäure aufgelöst. Dann werden bei 10 Grad Celsius 3,03 g (80 mmol) Natriumborhydrid zugesetzt. Die Mischung wird eine Stunde lang bei Raumtemperatur gerührt und dann drei Stunden lang auf 60 Grad Celsius erwärmt. Zur Aufbereitung wird die Mischung mit 200 ml Wasser verdünnt und mit 30 prozentiger Natronlauge auf einen pH-Wert von 5 eingestellt. Das ausgefallene Produkt wird abgesaugt, mit Wasser gewaschen und im Exsikkator über Calciumchlorid getrocknet. Man erhält 2,0 g (47 Prozent der Theorie) orangebraune Kristalle, die zwischen 134 und 137 Grad Celsius schmelzen.

Die in Beispiel 1 beschriebene Umsetzung kann auch mit homologen Carbonsäuren durchgeführt werden. So erhält man beim Einsatz von Isobuttersäure ein Produkt, welches identisch mit Beispiel 5, Stufe 2 und beim Einsatz von Pivalinsäure ein Produkt, welches identisch mit Beispiel 6, Stufe 2 ist.

**Beispiel 2:**

Darstellung von 2-Chlor-6-ethylamino-4-nitro-phenol

1. Stufe:

N-(3-Chlor-2-hydroxy-5-nitro-phenyl)acetamid

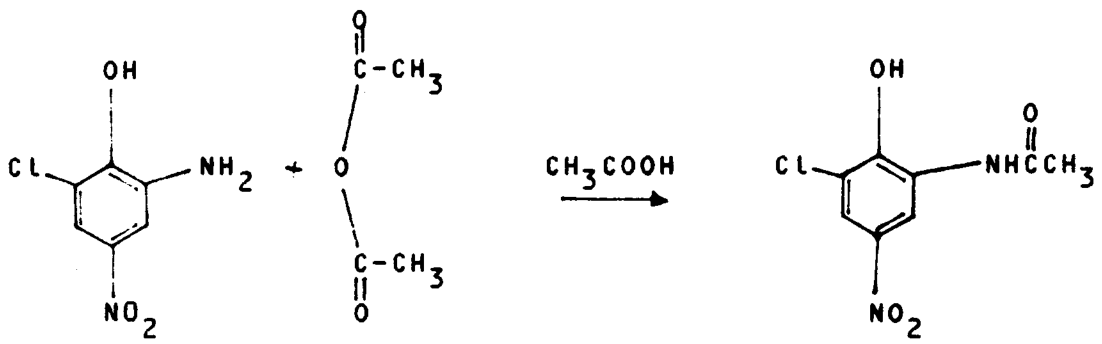


(188,54) (102,09) (230,6)

In einem Sulfierkolben werden 70 g (0,37 mmol) 2-Amino-6-chlor-4-nitro-phenol in 500 ml Essigsäure vorgelegt. Unter Rühren werden bei 25 Grad Celsius 100 ml (1,06 mol) Essigsäureanhydrid zugetropft. Die Mischung wird 5 Stunden lang bei Raumtemperatur gerührt. Danach wird die Mischung auf 2,5 kg Eiswasser gegossen und eine weitere Stunde lang gerührt. Das ausgefallene Produkt wird abgesaugt, mit Wasser gewaschen und aus 2 Teilen Ethanol und einem Teil1 Wasser umkristallisiert. Man erhält 59,9 g (69,9 Prozent der Theorie) ecrufarbenes Pulver, das einen Schmelzpunkt von über 200 Grad Celsius aufweist.

2. Stufe:

2-Chlor-6-ethylamino-4-nitro-phenol

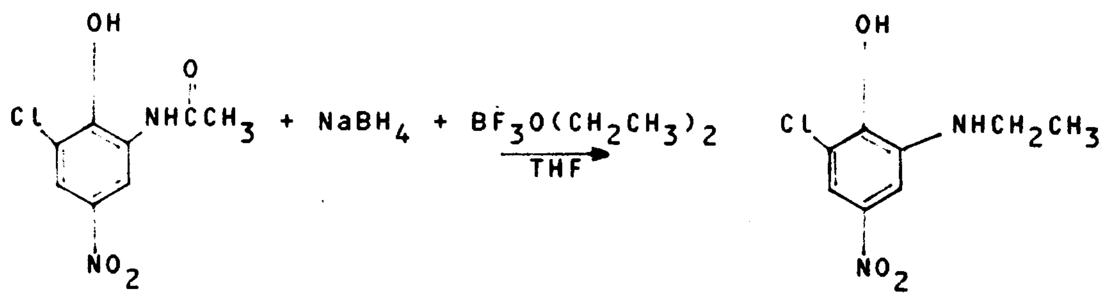


(230,6) (37,8) (141,93) (216,6)

Unter einer Stickstoffatmosphäre werden 50 g (0,22 mol) N-(3-Chlor-2-hydroxy-5-nitro-phenyl)acetamid aus Stufe 1 in 600 ml getrocknetem Tetrahydrofuran vorgelegt. Sodann werden unter Kühlung 18,9 g (0,5 mol) Natriumborhydrid zugegeben. Wenn die Wasserstoffentwicklung abgeklungen ist, werden unter Kühlen 85 ml (0,68 mol) Bortrifluoridethyletherat zugetropft. Die Reaktion ist exotherm und wird durch Kühlung auf 30 Grad Celsius gehalten. Nach Beendigung der Zugabe wird die Mischung 2 Stunden auf 60 Grad Celsius erwärmt. Anschließend wird die Mischung bei 0 Grad Celsius mit 185 ml eines Tetrahydrofuran-Wassergemisches (Verhältnis 1:1) hydrolysiert, mit 110 ml halbkonzentrierter Salzsäure angesäuert und eine Stunde

lang bei Raumtemperatur gerührt. Das Produkt wird isoliert durch Extraktion mit 3 x 250 ml Diethylether, nachdem die Reaktionsmischung mit Natronlauge auf einen pH-Wert von 5 eingestellt worden ist. Die Etherphase wird mit gesättigter Natriumchloridlösung gewaschen und über Magnesiumsulfat getrocknet. Das Rohprodukt wird aus Ethanol/Wasser (2:1) umkristallisiert. Man erhält 40,6 g (86,5 Prozent der Theorie) braune bis dunkelbraune Nadeln, welche einen Schmelzpunkt von 136 bis 138 Grad Celsius aufweisen.

| <u>CHN-Analyse:</u> | % C | % H | % N |
|---|---|---|---|
| $C_8H_9ClN_2O_3$ | | | |
| berechnet: | 44,36 | 4,19 | 12,93 |
| gefunden: | 44,33 | 4,25 | 12,93 |

Das so hergestellte Produkt ist identisch mit den Produkt, welches in Beispiel 1 beschrieben wurde.

**Beispiel 3:**

Darstellung von 2-Chlor-6-((2-hydroxyethyl)amino)-4-nitro-phenol (nicht-erfindungsgemäße Vergleichsverbindung)

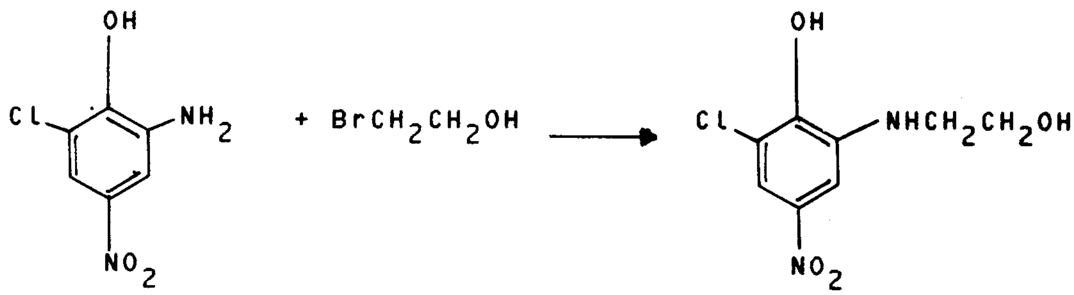

$C_6H_5ClN_2O_3$ (188,54) $C_2H_5BrO$ (124,97) $C_8H_9ClN_2O_4$ (232,6)

1,88 g (10 mmol) 2-Amino-6-chlor-4-nitro-phenol, 40 ml 2-Bromethanol und 1,0 g (10 mmol) Calciumcarbonat werden eine Stunde lang auf 120 Grad Celsius erwärmt. Danach wird die Mischung mit 300 ml Wasser verdünnt und mit Natronlauge auf einen pH-Wert von 12 eingestellt. Die Wasserphase wird mit 3 x 40 ml Diethylether extrahiert. Der Extrakt enthält Nebenprodukte und wird verworfen. Die Wasserphase wird mit Salzsäure auf einen pH-Wert von 5 eingestellt und mit 5 x 50 ml Diethylether extrahiert. Die Etherphase wird über Magnesiumsulfat getrocknet. Der nach dem Abdestillieren des Lösungsmittels erhaltene Rückstand wird zweimal aus Wasser umkristallisiert. Man erhält 0,68 g (29 Prozent der Theorie) eines orangefarbenen Pulvers, welches zwischen 142 und 144 Grad Celsius schmilzt.

| CHN-Analyse: | % C | % H | % N |
|---|---|---|---|
| $C_8H_9ClN_2O_4 \times 1/2\ H_2O$ | | | |
| berechnet: | 39,75 | 4,14 | 11,59 |
| gefunden: | 40,15 | 4,16 | 11,61 |

**Beispiel 4:**

Darstellung von 2-chlor-4-nitro-6-propylamino-phenol

1. Stufe:

N-(3-chlor-2-hydroxy-5-nitro-phenyl)propionsäureamid

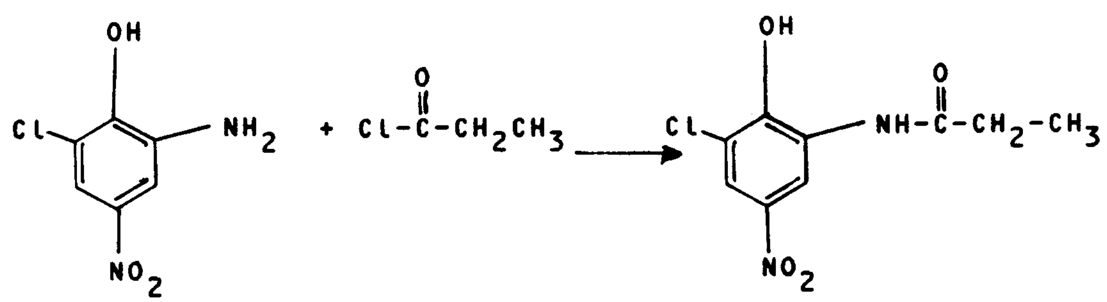

$C_6H_5ClN_2O_3$ (188,54) (92,53) $C_9H_9ClN_2O_4$ (244,64)

In einem Dreihalskolben mit Rückflußkühler, Thermometer und Tropftrichter werden unter Rühren 9,43 g (50 mmol) 2-Amino-6-chlor-4-nitro-phenol in 200 ml Dioxan aufgelöst. Dann werden bei Raumtemperatur 6,20 g (5,85 ml; 67 mmol) Propionsäurechlorid zugetropft. Die Mischung wird zwei Stunden lang bei Raumtemperatur gerührt und dann eine Stunde lang auf 90 Grad Celsius erwärmt. Danach wird die Mischung auf 500 g Eis gegossen. Der ausgeschiedene, beige Niederschlag wird abgesaugt und aus einer Ethanol/Wasser-Mischung (1 : 1) umkristallisiert.
Es werden 9,21 g (75 Prozent der Theorie) eines braunbeigen Produktes erhalten, das bei 167 Grad Celsius unter Zersetzung schmilzt.

| CHN-Analyse: | % C | % H | % N |
|---|---|---|---|
| $C_9H_9ClN_2O_4$ | | | |
| berechnet: | 44,19 | 3,71 | 11,45 |
| gefunden: | 44,24 | 3,77 | 11,30 |

2. Stufe:

2-Chlor-4-nitro-6-propylamino-phenol

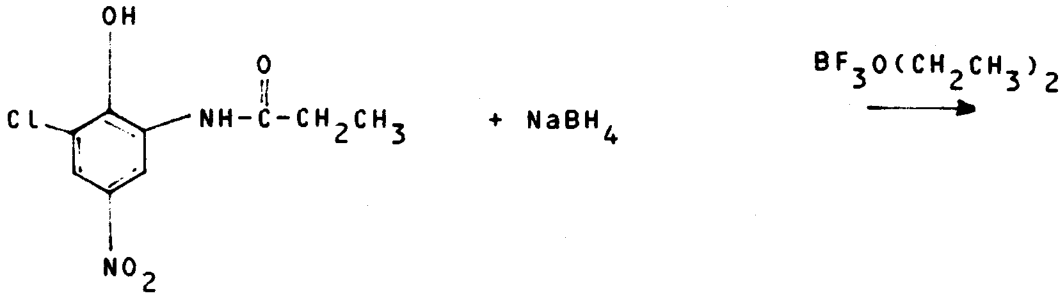


(244,64) (37,83) (141,93)

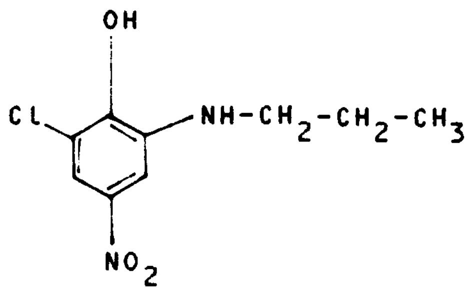

(230,65)

Unter einer Stickstoffatmosphäre werden 7,34 g (30 mmol) N-(3-Chlor-2-hydroxy-5-nitro-phenyl)-propionsäureamid aus Stufe 1 in 100 ml getrockentem Tetrahydrofuran vorgelegt. Nun werden unter Kühlen 2,27 g (60 mmol) Natriumborhydrid zugegeben. Wenn die Wasserstoffentwicklung abgeklungen ist, werden unter Kühlen 11,3 ml (90 mmol) Bortrifluoriddiethyletherat zugetropft. Die Reaktion ist exotherm und wird durch Kühlen auf 30 Grad Celsius gehalten. Sodann wird die Reaktionsmischung 2 Stunden lang auf 60 Grad Celsius erwärmt. Anschließend wird die Mischung auf 0 Grad Celsius abgekühlt und mit 15 ml Tetrahydrofuran-Wassermischung (Verhältnis 1:1) hydrolysiert, mit 10 ml halbkonzentrierter Salzsäure angesäuert und eine Stunde lang bei Raumtemperatur gerührt. Das Produkt wird durch Extraktion mit 3 x 100 ml Diethylether isoliert, nachdem mit Natronlauge ein pH-Wert von 5 eingestellt worden ist. Die Etherphase wird mit gesättigter Natriumchloridlösung gewaschen und über Magnesiumsulfat getrocknet. Das Rohprodukt wird aus 200 ml Cyclohexan umkristallisiert. Man erhält 4,00 g (58,5 Prozent der Theorie) eines gelborangen Pulvers, das zwischen 81 und 82,5 Grad Celsius schmilzt.

| CHN-Analyse: | % C | % H | % N |
|---|---|---|---|
| $C_9H_{11}ClN_2O_3$ | | | |
| berechnet: | 46,87 | 4,81 | 12,15 |
| gefunden: | 46,52 | 4,93 | 11,85 |

**Beispiel 5:**

Darstellung von 2-Chlor-6-((2-methylpropyl)amino)-4-nitro-phenol)

1. Stufe:

N-(3-Chlor-2-hydroxy-5-nitrophenyl)isobuttersäureamid

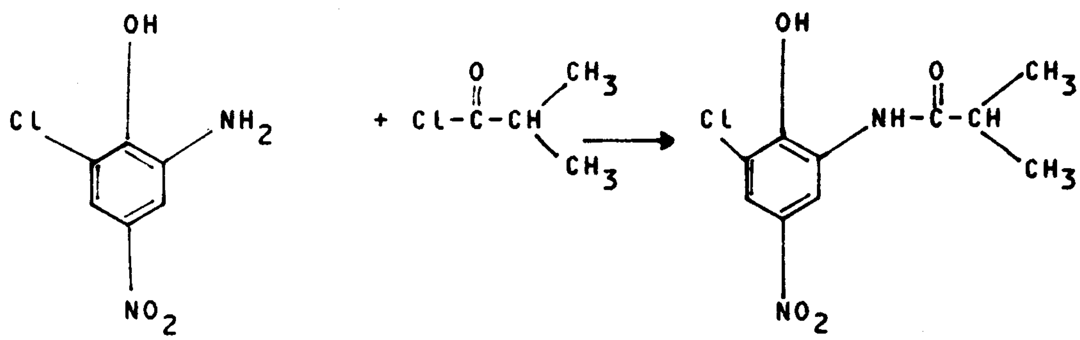

| | | |
|---|---|---|
| $C_6H_5ClN_2O_3$ | $C_4H_7ClO$ | $C_{10}H_{11}ClN_2O_4$ |
| (188,54) | (106,55) | (258,66) |

Ansatz:

9,43 g (50 mmol) 2-Amino-6-chlor-4-nitro-phenol
150 ml Tetrahydrofuran
7,14 g (67 mmol) Isobuttersäurechlorid
Die Verbindung wird analog wie im Beispiel 4, Stufe 1 beschrieben hergestellt.
Man erhält 10,1 g (75 Prozent der Theorie) eines beigen Produktes, das bei 158 Grad Celsius unter Zersetzung schmilzt.

| CHN-Analyse: | % C | % H | % N |
|---|---|---|---|
| $C_{10}H_{11}ClN_2O_4$ | | | |
| berechnet: | 46,44 | 4,29 | 10,93 |
| gefunden: | 46,46 | 4,38 | 10,59 |

2. Stufe:

2-Chlor-6-((2'-methylpropyl)amino)-4-nitro-phenol

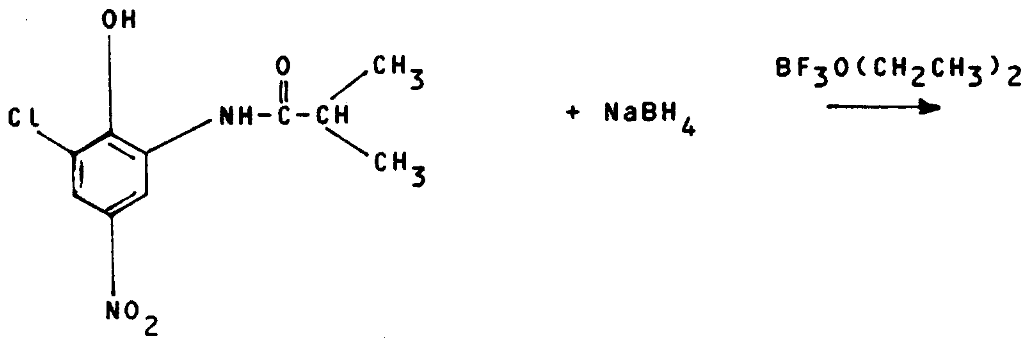


$C_{10}H_{11}ClN_2O_4$

(258,66) (37,83) (141,93)

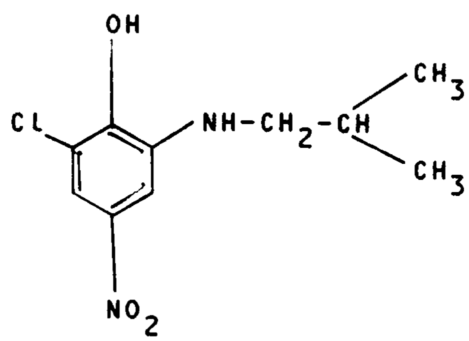

$C_{10}H_{13}ClN_2O_3$

(244,68)

Ansatz:

7,76 g (30 mmol) N-(3-Chlor-2-hydroxy-5-nitro-phenyl) isobuttersäureamid aus Stufe 1
100 ml Tetrahydrofuran
2,27 g (60 mmol) Natriumborhydrid
11,3 ml (90 mmol) Bortrifluorid-diethyletherat

Die Verbindung wird analog wie im Beispiel 4, Stufe 2 beschrieben dargestellt. Man erhält 6,0 g (82 Prozent der Theorie) eines orangen Produktes, das zwischen 115 und 116 Grad Celsius schmilzt.

| CHN-Analyse: | % C | % H | % N |
|---|---|---|---|
| $C_{10}H_{13}ClN_2O_3$ | | | |
| berechnet: | 49,09 | 5,36 | 11,45 |
| gefunden: | 48,90 | 5,45 | 11,27 |

**Beispiel 6:**

Darstellung von 2-Chlor-6-((2',2'-dimethylpropyl)amino)-4-nitro-phenol

1. Stufe:

N-(3-Chlor-2-hydroxy-5-nitro-phenyl)pivalinsäureamid

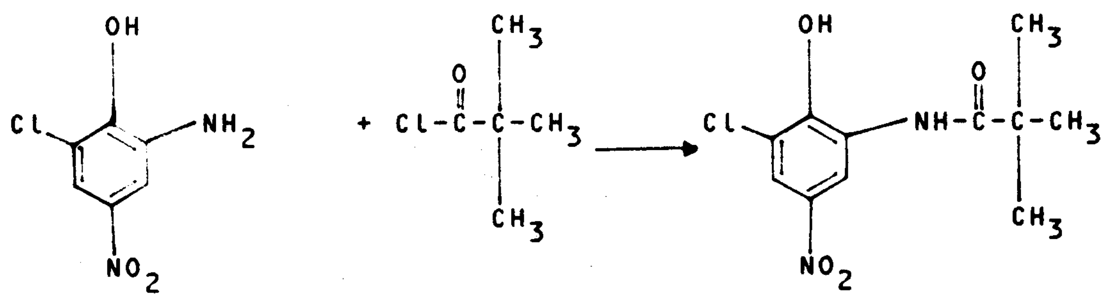

| | | |
|---|---|---|
| $C_6H_5ClN_2O_3$ | | $C_{11}H_{13}ClN_2O_4$ |
| (188,54) | (120,58) | (272,7) |

Ansatz:

9,43 g (50 mmol) 2-Amino-6-Chlor-4-nitro-phenol
150 ml Tetrahydrofuran
6,63 g (6.77 ml) (55 mmol) Pivalinsäurechlorid
Die Verbindung wird analog wie im Beispiel 4, Stufe 1 beschrieben dargestellt. Man erhält 10,7 g (78 Prozent der Theorie) beiges Pulver, das bei 140 Grad Celsius unter Zersetzung schmilzt.

2. Stufe:

2-Chlor-6-((2′,2′-dimethylpropyl)amino)-4-nitro-phenol

Reaktion:

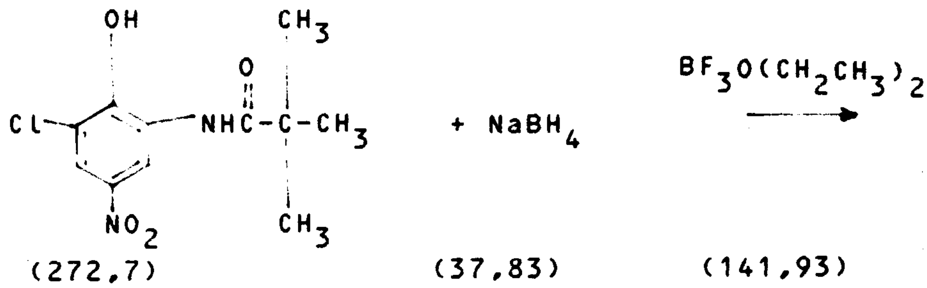


(272,7) (37,83) (141,93)

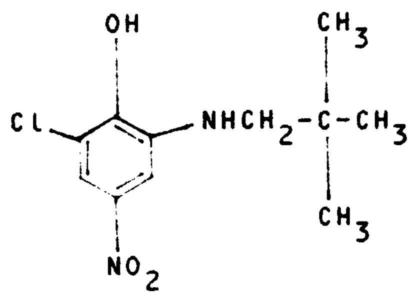

$C_{11}H_{15}ClN_2O_3$

(258,7)

Ansatz:

25 g ( 91,7 mmol) N-(3-Chlor-2-hydroxy-5-nitrophenyl) pivalinsäureamid aus Stufe 1
7,99 g (211 mmol) Natriumborhydrid
260 ml Tetrahydrofuran
40,68 g (36 ml) (286 mmol) Bortrifluorid-diethyletherat
Die Verbindung wird analog wie im Beispiel 4, Stufe 2 beschrieben dargestellt. Nach Kristallisation aus Ethanol/Wasser (1:1) werden 19,3 g (81 Prozent der Theorie) eines rötlich-braunen Pulvers erhalten, das zwischen 110 und 111 Grad Celsius schmilzt.

| CHN-Analyse: | % C | % H | % N |
|---|---|---|---|
| $C_{11}H_{15}ClN_2O_3$ | | | |
| berechnet: | 51,07 | 5,84 | 10,83 |
| gefunden: | 51,10 | 5,89 | 10,73 |

**Beispiel 7**

Darstellung von 2-Chlor-6-methylamino-4-nitro-phenol

1. Stufe

7-Chlor-5-nitro-benzoxazol

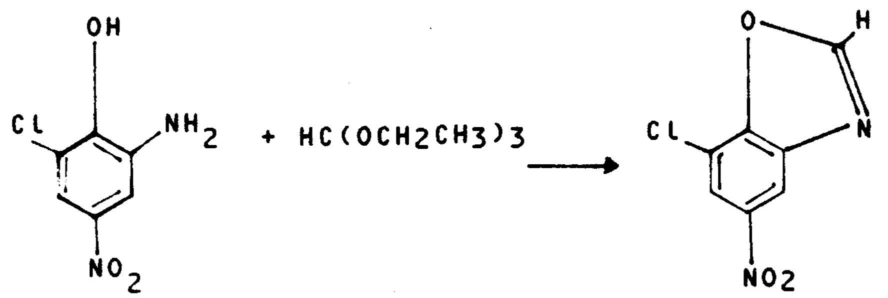


| | | |
|---|---|---|
| $C_6H_5ClN_2O_3$ | $C_7H_{16}O_3$ | $C_7H_3ClN_2O_3$ |
| (188,54) | (148,20) | (198,57) |

3,77 g (20 mmol) 2-Amino-6-chlor-4-nitro-phenol und 41,0 g (46 ml; 277 mmol) Orthoameisensäuretriethylester werden drei Stunden lang unter Rückfluß erwärmt (ölbadtemperatur 120 bis 130 Grad Celsius). Danach wird der nicht umgesetzte Orthoameisensäureester bei 149 Grad Celsius abdestilliert, und der Rückstand wird in 50 ml warmem Ethanol aufgenommen. Beim Abkühlen auf 0 Grad Celsius kristallisiert das Produkt aus. Es wird abgesaugt, mit wenig kaltem Ethanol gewaschen und getrocknet. Man erhält 2,7 g ( 68 Prozent der Theorie) gelb-beige Kristalle, die zwischen 121 und 122,5 Grad Celsius schmelzen.

| CHN-Analyse: | % C | % H | % N |
|---|---|---|---|
| $C_7H_3ClN_2O_3$ | | | |
| berechnet: | 42,34 | 1,52 | 14,11 |
| gefunden: | 42,19 | 1,66 | 14,02 |

2. Stufe:

2-Chlor-6-methylamino-4-nitro-phenol

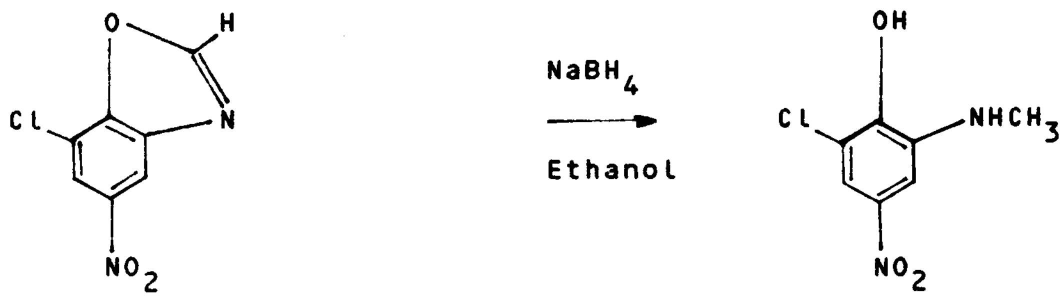


| | | |
|---|---|---|
| $C_7H_3ClN_2O_3$ | $Na_4BH$ | $C_7H_7ClN_2O_3$ |
| (198,57) | (37,83) | (202,60) |

2,0 g (10 mmol) des in Stufe 1 erhaltenen Produktes werden bei 0 Grad Celsius in Ethanol aufgeschlämmt und protionsweise mit 2,0 g (53 mmol) Natriumborhydrid versetzt. Die Mischung erwärmt sich und färbt sich rot. Sie wird 30 Minuten lang bei Raumtemperatur gerührt. Anschließend wird die Mischung filtriert und das Ethanol wird abdestilliert. Der Rückstand wird mit 200 g Wasser versetzt. Die rote Lösung wird mit zweinormaler Salzsäure auf einen pH-Wert von vier eingestellt. Das abgeschiedene beige Produkt wird abgesaugt und aus Wasser umkristallisiert. Es werden 2,0 g (50 Prozent der Theorie) einer gelben kristallinen Substanz erhalten, die zwischen 147 und 149 Grad Celsius schmilzt.

| CHN-Analyse: | % C | % H | % N | % Cl |
|---|---|---|---|---|
| $C_7H_7ClN_2O_3$ | | | | |
| berechnet: | 41,50 | 3,48 | 13,83 | 17,50 |
| gefunden : | 41,36 | 3,67 | 13,78 | 17,43 |

**Beispiele für Haarfärbemittel**

Beispiel 8 - 13: Haarfärbemittel

In einer Haarfärbelösung der Zusammensetzung

| | |
|---|---|
| 0,3 g | Farbstoff |
| 2,0 g | Laurylalkoholdiglykolethersulfat-Natriumsalz (28 prozentige wäßrige Lösung) |
| 2,0 g | Ammoniak (25 prozentige wäßrige Lösung) |
| 95,7 g | Wasser |
| 100,o g | |

wurden als Farbstoff 2-Amino-6-chlor-4-nitro-phenol, 6-Chlor-2-((2'-hydroxyethyl)amino)-4-nitro-phenol oder die Farbstoffe der Formel (I) worin R = $CH_3$, $C_2H_5$,$CH_2$-$CH(CH_3)_2$ oder $CH_2$-$C(CH_3)_3$ ist, eingesetzt. Gebleichtes menschliches Naturhaar wird 20 Minuten lang bei Raumtemperatur mit einer Lösung nach

Beispiel 8 - 13 behandelt. Danach wird das Haar mit Wasser gespült und getrocknet. Das Haar ist wie in der Tabelle 1 angegeben gefärbt:

Tabelle 1:

| Beispiel | Farbstoff | Haarfärbung |
|---|---|---|
| 8 | 2-Amino-6-chlor-4-nitro-phenol | orange |
| 9 | 6-chlor-2-((2'-hydroxyethyl)amino)-4-nitro-phenol | orange |
| 10 | Verbindung der Formel (I) (R = $CH_3$) | rot |
| 11 | Verbindung der Formel (I) (R = $C_2H_5$) | rot |
| 12 | Verbindung der Formel (I) (R = $CH_2-CH(CH_3)_2$) | leuchtend rot |
| 13 | Verbindung der Formel (I) (R = $CH_2-C(CH_3)_3$) | leuchtend rot |

**Beispiel 14:** Haarfärbelösung mit festigender Wirkung

| | |
|---|---|
| 0,1 g | 2-Chlor-6-ethylamino-4-nitro-phenol |
| 2,0 g | Polyvinylpyrrolidon |
| 0,1 g | Glyzerin |
| 40,0 g | Isopropanol |
| 57,8 g | Wasser |
| 100,0 g | |

Weiße menschliche Haare werden mit der Farbfestiger Lösung zur Frisur eingelegt und getrocknet. Das Haar ist rot gefärbt und gefestigt.

**Beispiel 15:** Oxidationshaarfärbemittel

| | |
|---|---|
| 0,40 g | 2,5-Diamino-toluolsulfat |
| 0,26 g | Resorcin |
| 0,40 g | 4-Amino-phenol |
| 0,13 g | 2,4-Diamino-anisol |
| 0,50 g | Farbstoff nach Beispiel 2 |
| 0,30 g | Ethylendiaminotetraessigsäure-dinatriumsalz |
| 0,30 g | Ascorbinsäure |
| 15,00 g | Cetylalkohol |
| 3,50 g | Laurylalkohol-diglykolethersulfat-Natriumsalz (28 prozentige wäßrige Lösung) |
| 6,00 g | Ammoniak, (25 prozentige wäßrige Lösung) |
| 73,21 g | Wasser, vollentsalzt |
| 100,00 g | |

50 ml des vorstehenden Haarfärbemittels werden kurz vor der Anwendung mit 50 ml Wasserstoffperoxidlösung (6 prozentig gemischt. Das Gemisch wird anschließend auf graue menschliche Haare aufgetragen und 30 Minuten bei einer Temperatur von 40 Grad Celsius einwirken gelassen. Nach dem Spülen des Haares mit Wasser und anschließendem Trocknen hat dieses eine Palisanderton angenommen. Das Haar ist gleichmäßig vom Haaransatz bis zu den Haarspitzen gefärbt.

## Patentansprüche

1. 2-Amino-6-chlor-4-nitro-phenolderivat der allgemeinen Formel (I)

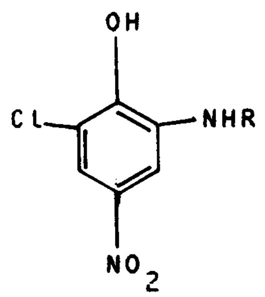

(I),

worin der Rest R einen geradkettigen oder verzweigten Alkylrest mit 1 bis 5 Kohlenstoffatomen bedeutet.

2. 2-Chlor-6-methylamino-4-nitro-phenol

3. 2-Chlor-6-ethylamino-4-nitro-phenol

4. 2-Chlor-4-nitro-6-propylamino-phenol

5. 2-Chlor-6-((2′-methylpropyl)amino)-4-nitro-phenol

6. 2-Chlor-6-((2′,2′-dimethylpropyl)amino)-4-nitrophenol

7. Verfahren zur Herstellung der Verbindung der allgemeinen Formel (I), dadurch gekennzeichnet, daß man zunächst 2-Amino-6-chlor-4-nitro-phenol mit einem Carbonsäurechlorid der Formel $R^1C(O)Cl$ oder einem Carbonsäureanhydrid der Formel $O(C(O)R^1)_2$, worin $R^1$ Wasserstoff oder einen geradkettigen oder verzweigten Alkylrest mit 1 bis 4 Kohlenstoffatomen darstellt, umsetzt und sodann das enstande-

ne, mit $R^1$ substituierte N-(3-Chlor-2-hydroxy-5-nitro-phenyl)carbonsäureamid mit Natriumborhydrid in Gegenwart von Bortrifluoridetherat zu der Verbindung der Formel (I) reduziert.

**8.** Verfahren zur Herstellung der Verbindung der allgemeinen Formel (I), dadurch gekennzeichnet, daß man zunächst 2-Amino-6-chlor-4-nitro-phenol in einer Carbonsäure der Formel $R^1C(O)OH$, worin $R^1$ Wasserstoff oder einen geradkettigen oder verzweigten Alkylrest mit 1 bis 4 Kohlenstoffatomen bedeutet, löst, sodann die so erhaltene Lösung mit Natriumborhydrid versetzt und anschließend auf 60 bis 70 Grad Celsius erhitzt.

**9.** Verfahren zur Herstellung der Verbindung der allgemeinen Formel (I), dadurch gekennzeichnet, daß man zunächst 2-Amino-6-chlor-4-nitro-phenol mit einem Orthocarbonsäureester der Formel $R^1C(OR^2)_3$, worin $R^1$ Wasserstoff oder einen geradkettigen oder verzweigten Alkylrest mit 1 bis 4 Kohlenstoffatomen darstellt und $R^2$ Methyl oder Ethyl bedeutet, umsetzt und das so erhaltene Benzoxazolderivat sodann mit Natriumborhydrid in alkoholischer Lösung reduziert.

**10.** Mittel zur Färbung von Haaren mit einem Farbstoffgehalt und für Haarfärbemittel üblichen Zusätzen, dadurch gekennzeichnet, daß es ein 2-Amino-6-chlor-4-nitro-phenolderivatder allgemeinen Formel (I)

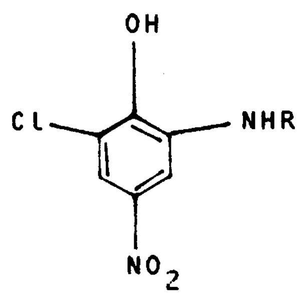

(I),

worin der Rest R einen geradkettigen oder verzweigten Alkylrest mit 1 bis 5 Kohlenstoffatomen bedeutet, oder dessen physiologisch verträgliches wasserlösliches Salz enthält.

**11.** Mittel nach Anspruch 10 dadurch gekennzeichnet, daß zusätzlich mindestens ein bekannter direktfärbender Haarfarbstoff enthalten ist.

**12.** Mittel nach Anspruch 11, dadurch gekennzeichnet, daß der direktfärbende Haarfarbstoff ausgewählt ist aus 2-Amino-4-nitro-phenol, 1-(2′-Hydroxyethyl)amino-2-amino-4-nitro-benzol, 2-Nitro-4-(2′-hydroxyethyl)amino-anilin, 1-Methylamino-2-nitro-4-di-(2′-hydroxyethyl)amino-benzol,1-(2′,3′-Dihydroxypropyl)-amino-2-nitro-4-(N-ethyl,N-(2″-hydroxyethyl)amino)benzol, 1-(2′,3′-Dihydroxypropyl)amino-2-nitro-4-dimethylamino-benzol, 1-(2′,3′-Dihydroxypropyl)amino-2-nitro-4-pyrrolidino-benzol, 1-(3′-Hydroxypropyl)-amino-2-nitro-4-di-(2″-hydroxyethyl)aminobenzol, 2,5-Bis(2′-hydroxyethyl)amino-nitrobenzol, Basic Violet 1 (C.I.42 535), Acid Brown 4 (C.I. 14 805), Disperse Violet 4 (C.I. 61 105), 1,4,5,8-Tetraamino-anthrachinon und 1,4-Diamino-anthrachinon.

**13.** Mittel nach Anspruch 11 oder 12, dadurch gekennzeichnet, daß es mindestens ein in der Kosmetik üblicherweise verwendetes synthetisches, natürliches oder modifiziertes natürliches Polymer enthält.

**14.** Mittel nach Anspruch 13, dadurch gekennzeichnet, daß das Polymer ausgewählt ist aus Polyvinylpyrrolidon, Polyvinylacetat, Polyvinylalkohol, Polyacrylsäure, Polymethacrylsäure, basischen Polymerisaten von Estern von Polyacrylsäure oder Polymethacrylsäure und Aminoalkoholen sowie dessen Salzen und Quaternisierungsprodukte, Polyacrylnitril, Polyvinylacetat, Polyvinylpyrrolidon-Vinylacetat sowie Chitosan und dessen Derivaten.

**15.** Mittel nach einem der Ansprüche 11 bis 13, dadurch gekennzeichnet, daß das 2-Amino-6-chlor-4-nitrophenolderivat der Formel (I) in einer Menge von 0,01 bis 2,0 Gewichtsprozent enthalten ist.

**16.** Mittel nach Anspruch 10, dadurch gekennzeichnet, daß es mindestens einen der bekannten Oxidationshaarfarbstoffe enthält.

17. Mittel nach Anspruch 16, dadurch gekennzeichnet, daß der Oxidationshaarfarbstoff ausgewählt ist aus p-Phenylendiamin, p-Toluylendiamin, p-Aminophenol, m-Phenylendiamin, Resorcin und m-Aminophenol.

18. Mittel nach Anspruch 16 oder 17, dadurch gekennzeichnet, daß das 2-Amino-6-chlor-4-nitro-phenolderivat der Formel (I) in einer Menge von 0,01 bis 4,0 Gewichtsprozent enthalten ist.

## Claims

1. 2-amino-6-chloro-4-nitro-phenol derivative of the general formula (I)

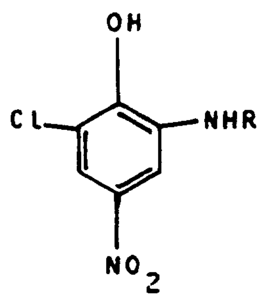

(I),

wherein the residue R is a straight-chain or branched alkyl residue with 1 to 5 carbon atoms.

2. 2-chloro-6-methylamino-4-nitro-phenol.

3. 2-chloro-6-ethylamino-4-nitro-phenol.

4. 2-chloro-4-nitro-6-propylamino-phenol.

5. 2-chloro-6-((2'-methylpropyl)amino)-4-nitro-phenol.

6. 2-chloro-6-((2'2'-dimethylpropyl)amino)-4-nitrophenol.

7. Method for preparing a compound of the general formula (I), characterized in that firstly 2-amino-6-chloro-4-nitro-phenol is reacted with a carboxylic acid chloride of the formula R'C(O)Cl or a carboxylic acid anhydride of the formula $O(C(O)R')_2$, wherein R' signifies hydrogen or a straight-chain or branched alkyl residue with 1 to 4 carbon atoms, and subsequently the resultant N-(3-chloro-2-hydroxy-5-nitro-phenyl)carboxylic acid amide substituted with R' is reduced with sodium borohydrate in the presence of boron trifluoride etherate to form the compound of formula (I).

8. Method for preparing a compound of the general formula (I), characterized in that firstly 2-amino-6-chloro-4-nitro-phenol is dissolved in a carboxylic acid of the formula R'C(O)OH, wherein R' signifies hydrogen or a straight-chain or branched alkyl residue with 1 to 4 carbon atoms, the solution obtained in this way is then mixed with sodium borohydride and then heated to 60 to 70 degrees Celsius.

9. Method for preparing a compound of the general formula (I), characterized in that firstly 2-amino-6-chloro-4-nitro-phenol is reacted with an orthocarboxylic acid ester of the formula $R^1C(OR^2)_3$, wherein $R^1$ signifies hydrogen or a straight-chain or branched alkyl residue with 1 to 4 carbon atoms and $R^2$ signifys methyl or ethyl, and the resultant benzoxazol derivative is subsequently reduced in an alcoholic solution with sodium borohydride.

10. Hair-colouring agent containing a colorant and additives conventional in hair colouring agents, characterized in that it contains a 2-amino-6-chloro-4-nitro-phenol derivative of the general formula (I)

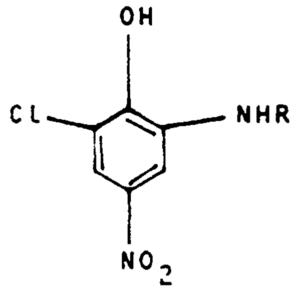

(I),

wherein the residue R signifies a straight-chain or branched alkyl residue with 1 to 5 carbon atoms, or physiologically compatible water-soluble salts thereof.

**11.** Agent according to claim 10, characterized in that it contains in addition at least one known direct-acting hair colorant.

**12.** Agent according to claim 11, characterized in that the direct-acting hair colorant is selected from 2-amino-4-nitrophenol,
1-(2'-hydroxyethyl)amino-2-amino-4-nitrobenzene,
2-nitro-4-(2'-hydroxyethyl)-aminoaniline,
1-methylamino-2-nitro-4-di-(2'-hydroxyethyl)amino-benzene 1-(2'3'-dihydroxypropyl)amino-2-nitro-4-(N-ethyl,
N-(2''-hydroxyethyl)amino)benzene,
1-(2'3'-dihydroxypropyl)amino-2-nitro-4-dimethylaminobenzene,
1-(2'3'-dihydroxypropyl)amino-2-nitro-4-pyrrolidinobenzene,
1-(3'-hydroxypropyl)amino-2-nitro-4-di(2''-hydroxyethyl) aminobenzene,
2,5-bis(2'-hydroxyethyl)amino-nitrobenzene, basic violet 1 (C.I. 42 535), acid brown 4 (C.I. 14 805), disperse violet 4 (C.I. 61 105), 1,4,5,8-tetraamino-anthraquinone and 1,4-diaminoanthraquinone.

**13.** Agent according to claim 11 or 12, characterized in that it contains at least one synthetic, natural or modified natural polymer which is conventionally used in cosmetics.

**14.** Agent according to claim 13, characterized in that the polymer is selected from polyvinylpyrrolidone, polyvinylacetate, polyvinylalcohol, polyacrylic acid, polymethacrylic acid, basic polymers of esters of polyacrylic acid or polymethacrylic acid and aminoalcohols as well as salts and quaternization products thereof, polyacrylonitrile, polyvinylacetate, polyvinylpyrrolidone-vinylacetate as well as chitosan and derivatives thereof.

**15.** Agent according to any one of claims 11 to 13, characterized in that the 2-amino-6-chloro-4-nitrophenol derivative of the formula (I) is present in an amount of 0.01 to 2.0 weightpercent.

**16.** Agent according to claim 10, characterized in that it contains at least one of the known oxidative hair colorants.

**17.** Agent according to claim 16, characterized in that the oxidative hair colorant is selected from p-phenylenediamine, p-toluenediamine, p-aminophenol, m-phenylenediamine, resorcinol and m-aminophenol.

**18.** Agent according to claim 16 or 17, characterized in that the 2-amino-6-chloro-4-nitrophenol derivative of formula (I) is contained in an amount of 0.01 to 4.0 weightpercent.

## Revendications

**1.** Dérivé de 2-amino-6-chloro-4-nitro-phénol de formule générale (I)

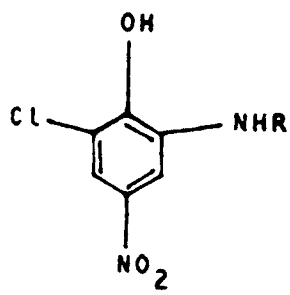

(I),

dans laquelle le reste R représente une chaine droite ou ramifiée comprenant 1 à 5 atomes de carbone.

**2.** 2-chloro-6-méthylamino-4-nitro-phénol.

**3.** 2-chloro-6-éthylamino-4-nitro-phénol.

**4.** 2-chloro-4-nitro-6-propylamino-phénol.

**5.** 2-chloro-6-(2-méthyl-propyl)-amino-4-nitro-phénol.

**6.** 2-chloro-6-((2', 2'-diméthylpropyl)-amino)-4-nitrophénol.

**7.** Procédé de fabrication du composé de formule générale (I) caractérisé en ce qu'on fait réagir d'abord du 2-amino-6-chloro-4-nitro-phénol avec un chlorure d'acide carboxylique de formule R'C(O)Cl ou un anhydride d'acide carboxylique de formule $O(C(O)R')_2$, R' représentant l'hydrogène ou un reste alkyle à chaine droite ou ramifiée comprenant de 1 à 4 atomes de carbone, puis on réduit l'amide d'acide N-(3-chloro-2-hydroxy-5-nitrophényl) carboxylique, substitué par R', obtenu, à l'aide d'hydrure de bore-sodium, en présence d'éthérate de trifluorure de bore, pour obtenir le dérivé de formule (I).

**8.** Procédé de préparation du composé de formule (I), caractérisé en ce qu'on dissout d'abord du 2-amino-6-chloro-4-nitro-phénol dans un acide carboxylique de formule $R^1C(O)OH$, dans laquelle $R^1$ représente l'hydrogène ou un reste alkyle à chaine droite ou ramifiée comprenant 1 à 4 atomes de carbone, puis on ajoute à la solution ainsi obtenue de l'hydrure de bore-sodium et enfin on chauffe de 60 à 70 °C.

**9.** Procédé de préparation du composé de formule général (I), caractérisé en ce qu'on fait d'abord réagir du 2-amino-6-chloro-4-nitro-phénol avec un ester de l'acide orthocarboxylique de formule $R^1C(OR^2)_3$, dans laquelle $R^1$ représente l'hydrogène ou un reste alkyle à chaine droite ou ramifiée comprenant de 1 à 4 atomes de carbone, et $R^2$ un radical méthyle ou éthyle, puis on réduit le dérivé benzoxazole ainsi obtenu par l'hydrure de bore-sodium en solution alcoolique.

**10.** Agent de teinture de cheveux contenant un colorant et des additifs usuels dans les teintures pour cheveux, caractérisé en ce qu'il contient un dérivé de 2-amino-6-chloro-4-nitro-phénol de formule générale (I)

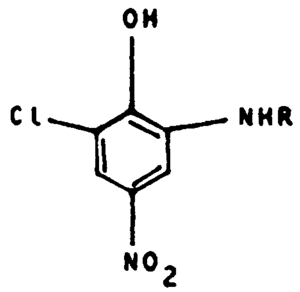

(I),

le reste R étant un reste alkyle à chaine droite ou ramifiée comprenant de 1 à 5 atomes de carbone, ou ses sels solubles dans l'eau, physiologiquement tolérés.

**11.** Agent selon la revendication 10, caractérisé en ce qu'il contient en outre au moins une teinture pour cheveux connue colorant directement.

**12.** Agent selon la revendication 11, caractérisé en ce que la teinture pour cheveux colorant directement est choisie parmi le 2-amino-4-nitro-phénol, le 1-(2'-hydroxléthyl)amino-2-amino-4-nitro-benzène, le 2-nitro-4-(2'-hydroxyéthyl)-amino-aniline, le 1-méthylamino-2-nitro-4-di-(2'-hydroxyéthyl)amino-benzène, le 1-(2',3'-dihydroxypropyl)amino-2-nitro-4-(N-éthyl,N-(2''-hydroxy-éthyl) amino)benzène, le 1-(2',3'-dihydroxypropyl)-amino-2-nitro-4-diméthyl-amino-benzène, le 1-(2',3'-dihydroxypropyl)amino-2-nitro-4-pyrrolidino-benzène, le 1-(3'-hydroxypropyl)amino-2-nitro-4-di-(2''-hydroxyéthyl)amino-benzène, le 2,5-bis(2'-hydroxy-éthyl)aminonitro-benzène, le basic violet 1 (C.I.42 535), l'acide Brown 4 (C.I. 14 805), le Diperse violet 4 (C.I.61 105), la 1,4,5,8-tétraamino-anthraquinone, et la 1,4-diamino-anthraquinone.

**13.** Agent selon la revendication 11 ou 12, caractérisé en ce qu'il contient au moins un polymère synthétique, naturel ou naturel modifié, utilisé courramment en cosmétologie.

**14.** Agent selon la revendication 13, caractérisé en ce que le polymère est choisi parmi la polyvinylpyrrolidone, l'acétate de polyvinyle, l'alcool polyvinylique, l'acide polyacrylique, l'acide polyméthacrylique, les polymères basiques d'esters d'acide polyacrylique ou polyméthacrylique et des aminoalcools, ainsi que leurs sels et leurs dérivés quaternisés, le polyacrylonitrile, l'acétate de polyvinyle, l'acétate de polyvinyl-polyvinylpyrrolidone, ainsi que le chitosane et ses dérivés.

**15.** Agent selon les revendications 11 à 13, caractérisé en ce que le dérivé 2-amino-6-chloro-4-nitro-phénol de formule (I) est utilisé à raison de 0,01 à 2,0%.

**16.** Agent selon la revendication 10, caractérisé en ce qu'il contient au moins un colorant de cheveux par oxydation connu.

**17.** Agent selon la revendication 16, caractérisé en ce que le colorant de cheveux par oxydation est choisi parmi la p-phénylènediamine, la p-toluylènediamine, le p-aminophénol, la m-phénylènediamine, la résorcine et le m-aminophénol.

**18.** Agent selon la revendication 16 ou 17, caractérisé en ce que le dérivé 2-amino-6-chloro-4-nitro-phénol est contenu à raison de 0,01 à 4,0%.